# EUROPEAN PATENT SPECIFICATION

(11) **EP 1 311 509 B1**
(45) Date of publication and mention of the grant of the patent: **24.03.2004**
(21) Application number: 01980251.1
(22) Date of filing: 17.08.2001
(51) Int. Cl.: C07D 471/04

(54) **PROCESS FOR THE PREPARATION OF IMIDAZOPYRIDINES**
PROZESS FÜR DIE HERSTELLUNG VON IMIDAZOPYRIDINEN
PROCEDE DE PREPARATION D'IMIDAZOPYRIDINES

(30) Priority: 17.08.2000 WO PCT/EP00/08021
(43) Date of publication of application: 21.05.2003
(73) Proprietor: Cilag AG, 8205 Schaffhausen (CH)
(72) Inventor: REY, Max, CH-8304 Wallisellen (CH); RÖSSLER, Armin, 78250 Tengen (DE); DERUNGS, Giusep, CH-8006 Zürich (CH); PAK, Jae, Kyoung, CH-8044 Zürich (CH)
(74) Representative: Braun, André
(86) International application number: PCT/EP2001/009519
(87) International publication number: WO 2002/014316

(56) References cited:
- EP-A- 0 050 563
- US-A- 4 794 185

## Description

The present invention relates to a process for preparing imidazopyridines of the general formula (1) 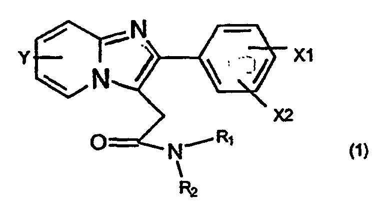 in which:
Y denotes hydrogen, a halogen or a C₁₋₄ alkyl group,
X₁ and X₂ denote, independently of each other, hydrogen, a halogen or a C₁₋₄ alkoxy, C₁₋₆ alkyl, CF₃, CH₃S, CH₃SO₂ or NO₂ group and
R₁ and R₂ denote, independently of each other, hydrogen or a C₁₋₅ alkyl group, with the proviso that R₁ and R₂ do not both denote hydrogen,
or salts thereof.

The products of this process are known to have useful pharmacological properties, e.g. as anxiolytics, see European Patent No. 0 050 563. A process for preparing compounds of formula 1 is described in US Patent No. 4,794,185, Dec. 12, 1988.

The present invention relates to a more efficient process for preparing compounds of formula (1).

In accordance with the present invention, compounds of the general formula (1) can be prepared by reacting a compound of the general formula (2) 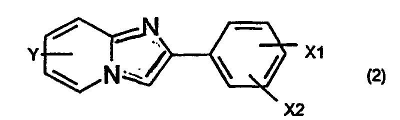 in which Y, X₁ and X₂, are as defined above
with a compound of the general formula (3) 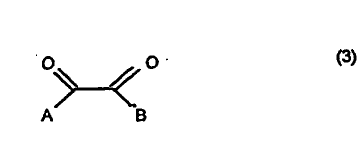 in which:
A denotes a halogen and B denotes a halogen, a C₁₋₄ alkoxy group or an NR₁R ₂ group in which R₁ and R₂ are as defined above
to form a compound of the general formula (4) 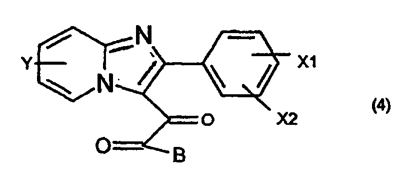 in which Y, X₁, X₂ and B are as defined above,
and, if B denotes a halogen or a C₁₋₄ alkoxy group, reacting the compound of the general formula (4) with a compound of the general formula (5) 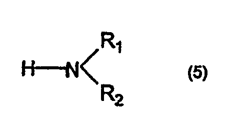 in which R₁ and R₂ are as defined above to form a compound of the general formula (6) 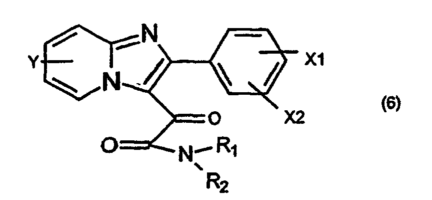 in which Y, X₁, X₂, R₁ and R₂ are as defined above.

To form a compound of formula (1), the compound of formula (6) can be treated with a reducing agent. If desired, the compound of formula (1) thus obtained is converted into a salt.

It will be appreciated that if in formula (3) B denotes an NR₁R ₂ group in which R₁ and R₂ are as defined above, compound (6) instead of compound (4) is formed directly by reaction of compound (2) with compound (3).

As set forth above compound (4) is prepared by reacting an imidazopyridine of formula (2) with an oxalic acid derivative of formula (3). This reaction is conveniently carried out in an aprotic organic solvent, for example n-hexane, cyclohexane, acetonitrile, acetone, ethylacetate, toluene, methyl tert. butyl ether or mixtures of these solvents, preferably a mixture of cyclohexane with toluene, at a temperature range from 0-100° C, preferably from 0-10°C, and in the presence of an organic base, for example tertiary alkylamines, pyridine or substituted pyridines, preferably pyridine. If in formula (3) B denotes a halogen or a C₁₋₄ alkoxy group, the product (4) thus obtained is subsequently reacted with a primary or secondary amine of formula (5), conveniently at a temperature range from 0-100°C, preferably from 30-40°C. If in formula (3) B denotes an NR₁R₂ group, the reaction of compound (2) with compound (3) directly yields a compound of formula (6) instead of compound (4), and no intervening treatment with a compound of formula (5) is necessary.

The compound of formula (6) thus obtained is then reacted with an appropriate reducing agent to form compound (1). This reaction is conveniently carried out in a polar aprotic solvent, for example pyridine, dimethylformamide or acetonitrile, preferably pyridine, in the presence of an organic acid, for example acetic acid, formic acid or toluenesulfonic acid, preferably acetic acid, and of an acylating agent, for example acetic anhydride or acetylchloride, preferably acetic anhydride, at a temperature range from 25-75°C, preferably from 50-55°C. A suitable reducing agent is, for example, Zn.

The compounds of the general formula (6) and their preparation also form part of the present invention.

The following examples illustrate the invention in greater detail.

### EXAMPLE 1

### Preparation of 6-methyl-N,N-dimethyl-2-(4-methylphenyl)imidazo[1,2-a]pyridine-3-glyoxyacetamide, compound (6)

To a slurry of 10.0 g (45 mmol) of 6-methyl-2-(4-methylphenyl)imidazo[1,2-a]pyridine in a mixture of 20.0 g of toluene and 28.0 g of cyclohexane were added 8.6 (0.068 mmol) of oxalylchloride within 15 minutes at 0-5°C. 3.6 g (45 mmol) of pyridine were added within 5 minutes at 0-5°C. The resulting slurry was heated to 65-70°C and stirred for 2 hours. Then it was cooled to 30-35°C and 8.4 g (187 mmol) of dimethylamine were introduced. To the slurry were added 26.0 g of water and 2.3 g of isopropanol. The product was isolated by filtration to afford the title compound in 80 % yield.

### EXAMPLE 2

### Preparation of N,N-dimethyl-2-[6-methyl-2-(4-methylphenyl)imidazo[1,2-a]pyridine-3-yl]acetamide, compound (1)

To a slurry of 150.0 g (0.467 mol) of 6-methyl-N,N-dimethyl-2-(4-methylphenyl)imidazo[1,2-a]pyridine-3-glyoxyacetamide and 105.0 g (1.605 mol) of zinc powder in 443.0 g of pyridine was added a solution of 94.0 g (0.920 mol) of acetic anhydride in 472.5 g of acetic acid within 20 - 25 minutes at a temperature below 45°C. The suspension was then heated to 50-55°C and stirred for 25-30 hours. Unreacted zinc was filtered off and the filtrate was subjected to a vacuum distillation. To the remaining oil 455.0 g of 25% aqueous ammonia solution were added. The precipitated solid was collected by filtration and purified by recrystallization in 800.0 g of methylisobutylketone. The title compound was afforded in 65.6 % yield.

## Claims

1. A process for preparing compounds of the general formula (6) 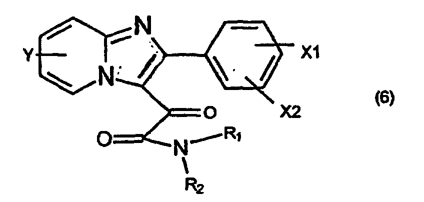 in which:
Y denotes hydrogen, a halogen or a C₁₋₄ alkyl group
X₁ and X₂ denote, independently of each other, hydrogen, a halogen or a C₁₋₄ alkoxy, C₁₋₆ alkyl, CF₃, CH₃S, CH₃SO₂ or NO₂ group and
R₁ and R₂ denote, independently of each other, hydrogen or a C₁₋₅ alkyl group, with the proviso that R₁ and R₂ do not both denote hydrogen,
which process comprises reacting a compound of the general formula (2) 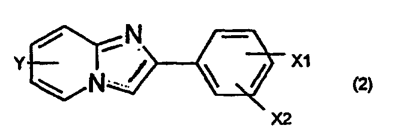 in which Y, X₁ and X₂, are as defined above
with a compound of the general formula (3) 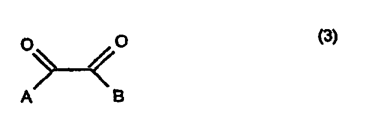 in which:
A denotes a halogen and B denotes a halogen, a C₁₋₄ alkoxy group or an NR₁R ₂ group in which R₁ and R₂ are as defined above
to form a compound of the general formula (4) 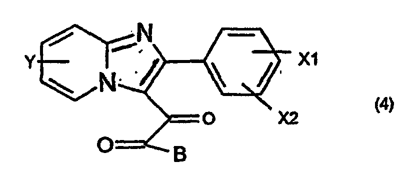 in which Y, X₁ X₂ and B are as defined above
and, if B denotes a halogen or a C₁₋₄ alkoxy group, reacting the compound of formula (4) with a compound of the general formula (5) 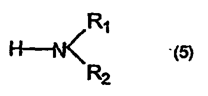 in which R₁ and R₂ are as defined above.

2. A compound of the general formula (6) 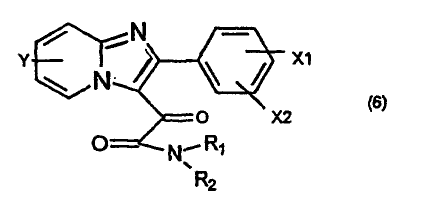 in which:
Y denotes hydrogen, a halogen or a C₁₋₄ alkyl group
X₁ and X₂ denote, independently of each other, hydrogen, a halogen or a C₁₋₄ alkoxy, C₁₋₆ alkyl, CF₃, CH₃S, CH₃SO₂ or NO₂ group and
R₁ and R₂ denote independently of each other, hydrogen or a C₁₋₅ alkyl group, with the proviso that R₁ and R₂ do not both denote hydrogen.

3. A process for preparing a compound of the general formula (1) 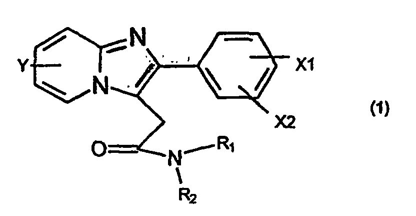 in which:
Y denotes hydrogen, a halogen or a C₁₋₄ alkyl group
X₁ and X₂ denote, independently of each other, hydrogen, a halogen or a C₁₋₄ alkoxy, C₁₋₆ alkyl, CF₃, CH₃S CH₃SO₂ or NO₂ group and
R₁ and R₂ denote independently of each other, hydrogen or a C₁₋₅ alkyl group, with the proviso that R₁ and R₂ do not both denote hydrogen,
or a salt thereof
which process comprises reducing a compound of the general formula (6) 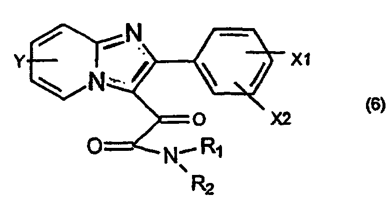 in which Y, X₁, X₂, R₁ and R₂ are as defined above
with an appropiate reducing agent and, if desired, converting the compound of formula (1) thus obtained into a salt.

4. A process according to claim 3 wherein the reducing agent is Zn .

5. A process according to claim 3 or claim 4 wherein the reduction is carried out in pyridine, dimethylformamide, dimethylacetamide, acetonitrile or a derivative of any of these, in the presence of acetic acid, formic acid or toluenesulfonic acid and of an acylating agent.

6. A process according to claim 5 wherein the acylating agent is acetic anhydride or acetylchloride.

## Patentansprüche

1. Verfahren zur Herstellung von Verbindungen der allgemeinen Formel (6) 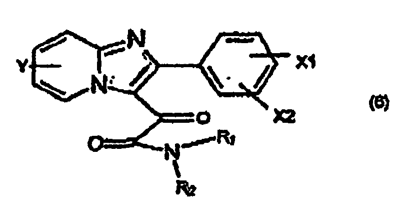 worin
Y Wasserstoff, ein Halogen oder eine C₁₋₄ Alkylgruppe bedeutet
X₁ und X₂ unabhängig voneinander Wasserstoff, ein Halogen oder eine C₁₋₄ Alkoxy-, C₁₋₆ Alkyl-, CF₃-, CH₃S-, CH₃SO₂- oder NO₂-Gruppe bedeuten und
R₁ und R₂ unabhängig voneinander Wasserstoff oder eine C₁₋₅ Alkylgruppe bedeuten, mit der Bedingung, dass R₁ und R₂ nicht beide Wasserstoff bedeuten,
wobei dieses Verfahren beinhaltet, dass man eine Verbindung der allgemeinen Formel (2) 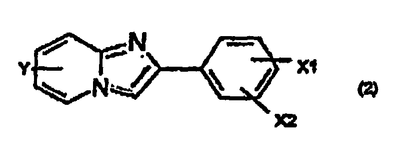 worin Y, X₁ und X₂ die oben angegebene Bedeutung haben
mit einer Verbindung der allgemeinen Formel (3) 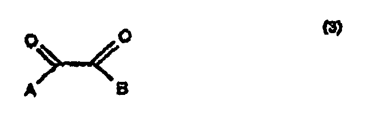 umsetzt,
worin:
A ein Halogen und B ein Halogen, eine C₁₋₄ Alkoxygruppe oder eine NR₁R₂-Gruppe bedeuten, in welcher R₁ und R₂ die oben angegebene Bedeutung haben,
um eine Verbindung der allgemeinen Formel (4) zu bilden 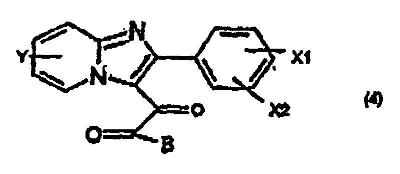 in welcher Y, X₁, X₂ und B die oben angegebene Bedeutung haben
und, wenn B ein Halogen oder eine C₁₋₄ Alkoxygruppe bedeutet, man die Verbindung der Formel (4) mit einer Verbindung der allgemeinen Formel (5) 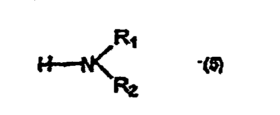 worin R₁ und R₂ die oben angegebene Bedeutung haben
umsetzt.

2. Verbindung der allgemeinen Formel (6) 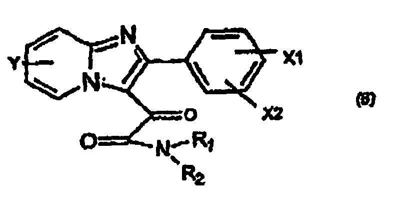 worin
Y Wasserstoff, ein Halogen oder eine C₁₋₄ Alkylgruppe bedeutet
X₁ und X₂ unabhängig voneinander Wasserstoff, ein Halogen oder eine C₁₋₄ Alkoxy-, C₁₋₆ Alkyl-, CF₃-, CH₃S-, CH₃SO₂- oder NO₂-Gruppe bedeuten und
R₁ und R₂ unabhängig voneinander Wasserstoff oder eine C₁₋₅ Alkylgruppe bedeuten, mit der Bedingung, dass R₁ und R₂ nicht beide Wasserstoff bedeuten.

3. Verfahren zur Herstellung einer Verbindung der allgemeinen Formel (1) 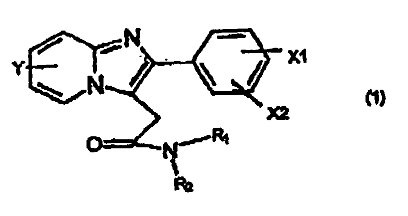 worin
Y Wasserstoff, ein Halogen oder eine C₁₋₄ Alkylgruppe bedeutet
X₁ und X₂ unabhängig voneinander Wasserstoff, ein Halogen oder eine C₁₋₄ Alkoxy-, C₁₋₆ Alkyl-, CF₃-, CH₃S-, CH₃SO₂- oder NO₂-Gruppe bedeuten und
R₁ und R₂ unabhängig voneinander Wasserstoff oder eine C₁₋₅ Alkylgruppe bedeuten, mit der Bedingung, dass R₁ und R₂ nicht beide Wasserstoff bedeuten,
oder ein Salz davon,
wobei dieses Verfahren beinhaltet, dass man eine Verbindung der allgemeinen Formel (6) 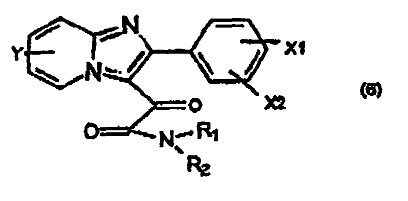 in welcher Y, X₁, X₂, R₁ und R₂ die oben angegebene Bedeutung haben
mit einem geeigneten Reduktionsmittel reduziert und, falls erwünscht, die so erhaltene Verbindung der Formel (1) in ein Salz umwandelt.

4. Verfahren nach Anspruch 3, worin das Reduktionsmittel Zn ist.

5. Verfahren nach Anspruch 3 oder Anspruch 4, worin die Reduktion in Pyridin, Dimethylformamid, Dimethylacetamid, Acetonitril oder einem Derivat dieser Verbindungen durchgeführt wird, in Gegenwart von Essigsäure, Ameisensäure oder Toluensulfonsäure und eines Acylierungsmittels.

6. Verfahren nach Anspruch 5, worin das Acylierungsmittel Essigsäureanhydrid oder Acetylchlorid darstellt.

## Revendications

1. Un procédé pour la préparation de composés de la formule générale (6) 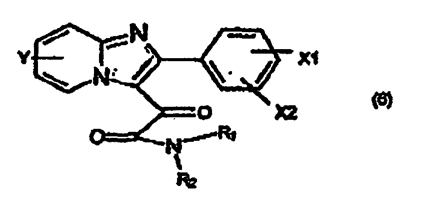 dans laquelle :
Y indique de l'hydrogène, un halogène ou un groupe C₁₋₄ alkyle,
X₁ et X₂ indiquent, indépendant l'un de l'autre, de l'hydrogène, un halogène ou un groupe C₁₋₄ alkoxy, C₁₋₆ alkyle, CF₃ CH₃S, CH₃SO₂ ou NO₂ et
R₁ et R₂ indiquent, indépendant l'un de l'autre, de l'hydrogène ou un groupe C₁₋₅ alkyle, à condition que R₁ et R₂ ne soient pas tous les deux de l'hydrogène,
ledit procédé comprenant la réaction d'un composé de la formule générale (2) 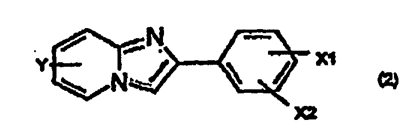 dans laquelle Y, X₁ et X₂ sont comme définis ci-dessus,
avec un composé de la formule générale (3) 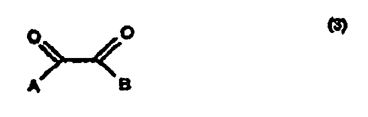 dans laquelle :
A indique un halogène et B indique un halogène, un groupe C₁₋₄ alkoxy
ou un groupe NR₁R₂ où R₁ et R₂ sont comme définis ci-dessus
afin de former un composé de la formule générale (4) 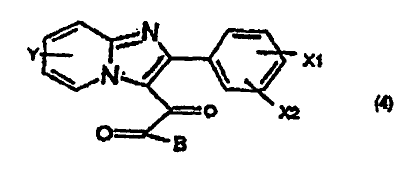 dans laquelle Y, X₁ X₂ et B sont comme définis ci-dessus
et si B indique un halogène ou un groupe C₁₋₄ alkoxy, on fait réagir le composé de la formule (4) avec un composé de la formule générale (5) 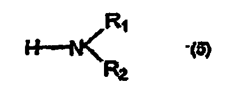 dans laquelle R1 et R2 sont comme définis ci-dessus.

2. Un composé de la formule générale (6) 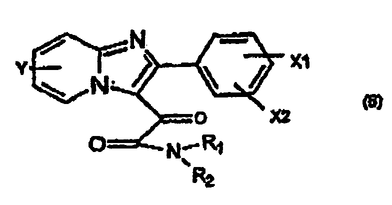 dans laquelle :
Y indique de l'hydrogène, un halogène ou un groupe C₁₋₄ alkyle,
X₁ et X₂ indiquent, indépendant l'un de l'autre, de l'hydrogène, un halogène ou un groupe C₁₋₄ alkoxy, C₁₋₆ alkyle, CF₃ CH₃S, CH₃SO₂ ou NO₂ et
R₁ et R₂ indiquent, indépendant l'un de l'autre, de l'hydrogène ou un groupe C₁₋₅ alkyle, à condition que R₁ et R₂ ne soient pas tous les deux de l'hydrogène.

3. Un procédé pour la préparation d'un composé de la formule générale (1) 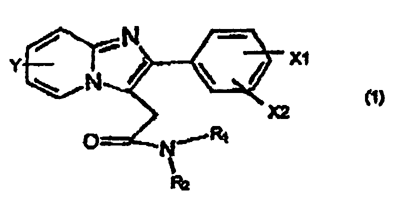 dans laquelle :
Y indique de l'hydrogène, un halogène ou un groupe C₁₋₄ alkyle,
X₁ et X₂ indiquent, indépendant l'un de l'autre, de l'hydrogène, un halogène ou un groupe C₁₋₄ alkoxy, C₁₋₆ alkyle, CF₃ CH₃S, CH₃SO₂ ou NO₂ et
R₁ et R₂ indiquent, indépendant l'un de l'autre, de l'hydrogène ou un groupe C₁₋₅ alkyle, à condition que R₁ et R₂ ne soient pas tous les deux de l'hydrogène,
ou un de leurs sels
ledit procédé comprenant la réduction d'un composé de la formule générale (6) 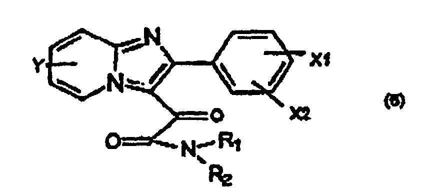 dans laquelle Y, X₁, X₂, R₁ et R₂ sont comme définis ci-dessus
avec un agent de réduction approprié et, si désiré, la transformation du composé de la formule (1) ainsi obtenu en un sel.

4. Un procédé selon la revendication 3, où l'agent de réduction est Zn.

5. Un procédé selon la revendication 3 ou la revendication 4 où la réduction est réalisée dans du pyridine, diméthylformamide, diméthylacétamide, acétonitrile ou un de leurs dérivés, en présence d'acide acétique, acide formique ou acide toluénesulfonique et d'un agent d'acylation.

6. Un procédé selon la revendication 5, où l'agent d'acylation est de l'anhydride acétique ou acétylchlorure.
